# FASCICULE DE BREVET EUROPEEN

(11) **EP 3 709 920 B1**
(45) Date de publication et mention de la délivrance du brevet: **24.04.2024**
(21) Numéro de dépôt: 18807567.5
(22) Date de dépôt: 12.11.2018
(51) Int. Cl.: A61B 18/22, A61M 25/01, A61B 18/24

(54) **ENSEMBLE ET DISPOSITIF DE TRAITEMENT ENDOVEINEUX**
ANORDNUNG UND VORRICHTUNG ZUR ENDOVENÖSEN BEHANDLUNG
ENDOVENOUS TREATMENT ASSEMBLY AND DEVICE

(30) Priorité: 13.11.2017 FR 1760627
(43) Date de publication de la demande: 23.09.2020
(73) Titulaire: LSO MEDICAL, 59120 Loos (FR)
(72) Inventeur: ROCHON, Philippe, 59182 Loffre (FR)
(74) Mandataire: Matkowska, Franck
(86) Numéro de dépôt international: PCT/EP2018/080857
(87) Numéro de publication internationale: WO 2019/092228

(56) Documents cités:
- WO-A1-99/15237
- US-A1- 2005 131 400
- US-A1- 2010 069 833
- US-B1- 6 485 482

## Description

### Domaine technique

La présente invention concerne le domaine du traitement endoveineux, par délivrance dans la veine d'une dose de traitement, au moyen d'un élément filaire souple. La dose de traitement peut, de manière non limitative et non exhaustive, être une dose d'énergie, délivrée par exemple sous la forme d'un rayonnement électromagnétique, au moyen d'ondes sonores ou ultrasonores, d'ondes radiofréquences, ou être une dose d'énergie thermique délivrée par rayonnement et/ou par contact, ou une dose d'un produit permettant un traitement de la veine. L'élément filaire souple peut être creux ou plein, et peut notamment, de manière non limitative et non exhaustive, être une fibre optique, un élément filaire de type câble ou une sonde souple ou une canule souple.

### Art antérieur

Dans le domaine du traitement endoveineux, il est connu de traiter une veine en délivrant dans la veine des doses de traitement au moyen d'un élément filaire souple, qui est inséré longitudinalement dans la veine, et dont le mouvement de retrait doit être contrôlé en cours de traitement. Plus particulièrement, dans le domaine de la thérapie par laser endoveineux, plus connue sous l'acronyme EVLT, il est usuel de traiter une veine au moyen d'un laser endoveineux (par exemple pour l'occlusion des varices saphènes par laser endoveineux), dont l'élément filaire souple est une fibre optique utilisée pour émettre un rayonnement électromagnétique dans la veine. Pour d'autres types de traitement endoveineux, l'élément filaire souple peut, de manière non limitative et non exhaustive, également être un câble ou une sonde souple ou une canule souple.

Des exemples de dispositif de traitement endoveineux sont décrits par exemple dans les publications suivantes : US 2005/0131400, US2008/0097224, US2008/0097408, US 6 814 727, US 2010/069833, US 6 485 482.

De manière usuelle, le retrait de l'élément filaire souple inséré longitudinalement dans la veine, et par exemple de la fibre optique, peut être commandé en cours de traitement par le praticien au moyen d'un système d'entraînement motorisé, encore appelé système de retrait, permettant de tirer longitudinalement sur la partie extrême arrière (la plus éloignée du corps du patient) de l'élément filaire souple en contrôlant la vitesse de retrait. Ce retrait peut selon le cas être effectué de manière continue ou pas à pas. Les systèmes de retrait motorisés couramment utilisés comportent des moyens d'entrainement sous la forme d'au moins une paire de rouleaux (ou galets) d'entraînement entre lesquels passe l'élément filaire souple et qui permettent l'entraînement de cet élément filaire par friction.

La mise en oeuvre d'un système d'entraînement dans lequel l'élément filaire souple peut être entraîné par friction ou autre, en étant en prise avec des moyens d'entraînement, tels que par exemple (mais pas exclusivement) des galets ou rouleaux d'entraînement, pour son entraînement par lesdits moyens d'entraînement dans le sens de sa longueur au moins dans une première direction, et de préférence également dans la direction inverse, présente l'avantage de permettre un déplacement de l'élément filaire souple sur une course très importante, et notamment sur une course pouvant le cas échéant atteindre quasiment toute la longueur de cet élément filaire souple.

Les éléments filaires souple de délivrance de doses, tels qu'une fibre optique sont généralement des consommables à usage unique, qui sont jetés une fois qu'ils ont été utilisés.

En pratique, à chaque traitement le praticien retire de son emballage stérile l'élément filaire souple de délivrance de doses, tel que par exemple une fibre optique et doit correctement positionner à la main une portion de cet élément filaire souple entre les rouleaux d'entraînement du système d'entraînement.

Ces opérations de manutention et de positionnement de l'élément filaire souple de délivrance de doses par rapport aux rouleaux d'entraînement du système d'entraînement sont contraignantes et fastidieuses pour le praticien et peuvent en outre occasionner une détérioration accidentelle de l'élément filaire souple de délivrance de doses, notamment lorsqu'il s'agit d'une fibre optique. En outre, il peut arriver que le praticien ne positionne pas correctement dès sa première tentative l'élément filaire souple de délivrance de doses par rapport aux rouleaux d'entraînement du système d'entraînement et qu'il doive recommencer plusieurs fois.

Par ailleurs, il est intéressant de pouvoir mettre en oeuvre un élément filaire ayant une longueur importante, de manière à pouvoir facilement déporter, en dehors du champ opératoire stérile, le système d'entraînement motorisé et le système de délivrance de doses de traitement, tel que par exemple la source laser dans le cas d'un laser endoveineux. Or cette longueur importante de l'élément filaire pose un problème d'encombrement, notamment lors des opérations de retrait de l'élément filaire.

On a également déjà proposé dans la demande de brevet internationale WO99/15237 une solution technique mettant en oeuvre une pièce de positionnement et de guidage d'une fibre optique par rapport à un système d'entraînement de la fibre optique. Dans cette solution, la fibre optique n'est pas entraînée par friction, mais est fixée à un piston qui est monté à l'intérieur d'une pièce de positionnement et de guidage. La pièce de positionnement et de guidage est adaptée pour être fixée de manière amovible sur un système d'entraînement comportant une tige mobile en translation, de telle sorte que ladite tige peut pousser sur le piston, provoquant ainsi le déplacement de la fibre optique fixée au piston, dans le sens de sa longueur dans une première direction. Un ressort de rappel est en outre monté dans la pièce de positionnement et guidage et permet un rappel en position du piston et de ce fait un déplacement de la fibre optique fixée au piston dans la direction inverse.

Cette solution décrite dans la demande de brevet internationale WO99/15237 ne peut pas être utilisée avec un système d'entraînement dans lequel l'élément filaire de délivrance de doses de traitement peut être entraîné par friction ou autre, en étant en prise avec des moyens d'entraînement, tels que par exemple (mais pas exclusivement) des galets ou rouleaux d'entraînement, pour son entraînement par lesdits moyens d'entraînement dans le sens de sa longueur au moins dans une première direction, et de préférence également dans la direction inverse.

En outre de manière désavantageuse, dans la solution décrite dans la demande de brevet internationale WO99/15237, la course du déplacement de l'élément filaire de délivrance de doses de traitement est très faible et est limitée à la course du piston d'entraînement.

### Objectif de l'invention

Un objectif de l'invention est d'une manière générale de proposer dans le domaine du traitement endoveineux une nouvelle solution technique qui permet de simplifier et de fiabiliser les opérations de manipulation et de positionnement de l'élément filaire souple de délivrance des doses de traitement par rapport à des moyens d'entraînement du système d'entraînement d'un dispositif endoveineux.

Un objectif optionnel plus particulier est de proposer une nouvelle solution technique ne présentant pas l'inconvénient susvisé de limitation de la course de déplacement de l'élément filaire de délivrance de doses de traitement, inhérent à la solution décrite dans la demande de brevet internationale WO99/15237.

### Résumé de l'invention

L'invention est définie dans les revendications attenantes.

L'invention a ainsi pour objet un ensemble de traitement endoveineux comportant un élément filaire de délivrance de doses de traitement, qui est souple et apte à être inséré, sur une partie de sa longueur, longitudinalement dans une veine, et une pièce de positionnement et guidage, qui est solidaire de l'élément filaire de délivrance de doses de traitement de manière à permettre la manipulation de l'élément filaire de délivrance de doses de traitement au moyen de cette pièce de positionnement et guidage ; ladite pièce de positionnement et guidage comporte au moins un premier moyens de guidage en translation d'une première portion de l'élément filaire de délivrance de doses de traitement dans le sens de sa longueur, l'élément filaire de délivrance de doses de traitement pouvant coulisser dans le sens de sa longueur par rapport audit premier moyen de guidage en translation de ladite pièce de positionnement et guidage et ladite pièce de positionnement et guidage comportant des premiers moyens d'assemblage mécanique permettant son montage amovible par rapport à des moyens d'entraînement d' un système d'entraînement, de telle sorte que ladite première portion de l'élément filaire de délivrance de doses de traitement peut être positionnée et guidée au moyen de cette pièce de positionnement et guidage en étant en prise avec des moyens d'entraînement du système d'entraînement pour l'entraînement de l'élément filaire de délivrance de doses de traitement dans le sens de sa longueur au moins dans une première direction.

L'invention a également pour objet un ensemble de traitement endoveineux comportant un élément filaire de délivrance de doses de traitement, qui est souple et apte à être inséré, sur une partie de sa longueur, longitudinalement dans une veine, et une pièce de positionnement et guidage, qui est solidaire de l'élément filaire de délivrance de doses de traitement de manière à permettre la manipulation de l'élément filaire de délivrance de doses de traitement au moyen de cette pièce de positionnement et guidage ; l'élément filaire de délivrance de doses de traitement peut coulisser dans le sens de sa longueur par rapport à ladite pièce de positionnement et guidage ; ladite pièce de positionnement et guidage comporte au moins un premier moyen de guidage en translation d'une première portion de l'élément filaire de délivrance de doses de traitement dans le sens de sa longueur et des premiers moyens d'assemblage mécanique permettant son montage amovible par rapport à un système d'entraînement comportant au moins une paire de galets d'entraînement, de telle sorte que ladite première portion de l'élément filaire de délivrance de doses de traitement peut être positionnée et guidée au moyen de cette pièce de positionnement et guidage entre les galets d'entraînement, pour l'entraînement de l'élément filaire de délivrance de doses de traitement dans le sens de sa longueur au moins dans une première direction .

Plus particulièrement, l'ensemble de l'invention peut comporter les caractéristiques additionnelles et optionnelles suivantes, prises isolément, ou en combinaison les unes avec les autres :
- ladite première portion de l'élément filaire de délivrance de doses de traitement est accessible ou peut être rendue accessible.
- ladite première portion de l'élément filaire de délivrance de doses de traitement peut être positionnée et guidée au moyen de la pièce de positionnement et guidage de manière à pouvoir être en prise avec des moyens d'entraînement du système d'entraînement pour l'entraînement de l'élément filaire de délivrance de doses de traitement dans le sens de sa longueur au moins dans une première direction, avec un déplacement de l'élément filaire de délivrance de doses de traitement dans le sens de sa longueur par rapport auxdits moyens d'entraînement.
- les premiers moyens d'assemblage mécanique permettent un montage rapide et sans outil de la pièce de positionnement et guidage.
- l'ensemble comporte des deuxièmes moyens de guidage d'une deuxième portion de l'élément filaire de délivrance de doses de traitement.
- les deuxièmes moyens de guidage guident en translation cette deuxième portion de l'élément filaire de délivrance de doses de traitement en lui faisant effectuer au moins un quart de tour et de préférence au moins un demi-tour.
- les deuxièmes moyens de guidage font partie intégrante de la pièce de positionnement et guidage.
- l'élément filaire de délivrance de doses de traitement comporte une troisième portion, qui s'étend jusqu'à l'extrémité arrière de l'élément filaire de délivrance de doses de traitement, et qui n'est pas solidaire de la pièce de positionnement et guidage ou qui est solidaire de la pièce de positionnement et guidage mais peut être séparée de cette pièce de positionnement et guidage.
- l'ensemble comporte un coupleur qui est fixé à l'extrémité arrière de l'élément filaire de délivrance de doses de traitement et qui permet de connecter l'élément filaire de délivrance de doses de traitement à une source de doses de traitement.
- la pièce de positionnement et guidage est une pièce monobloc, et plus particulièrement une pièce moulée.
- la pièce de positionnement et guidage est constituée par un assemblage monolithique.
- l'ensemble comporte un guide souple enfilé sur une portion avant de l'élément filaire de délivrance de doses de traitement, ledit l'élément filaire de délivrance de doses de traitement étant apte à coulisser longitudinalement par rapport au guide souple.
- l'extrémité arrière du guide souple est solidaire de la pièce de positionnement et guidage, de manière à bloquer axialement la partie arrière du guide souple au moins dans la première direction d'entrainement de l'élément filaire de délivrance de doses de traitement, et de préférence également dans la direction) opposée à la direction d'entraînement de l'élément filaire de délivrance de doses de traitement.
- l'ensemble comporte un support de stockage, sur lequel est enroulée tout ou partie de la portion avant de l'élément filaire de délivrance de doses de traitement qui s'étend depuis l'extrémité avant de l'élément filaire de délivrance de doses jusqu'à ladite première portion de l'élément filaire de délivrance de doses de traitement.
- le guide souple et la partie de l'élément filaire de délivrance de doses de traitement enfilée dans le guide souple sont enroulés sur le support de stockage.
- le support de stockage et la pièce de positionnement et guidage sont séparés ou dans lequel le support de stockage et la pièce de positionnement et guidage sont assemblées et séparables l'un de l'autre.
- l'élément filaire de délivrance de doses de traitement est une fibre optique.
- L'ensemble comporte en outre un système de maintien qui permet de maintenir temporairement la partie extrême avant du guide souple par rapport au corps d'un patient, à proximité de la zone d'insertion de l'élément filaire de délivrance de doses de traitement.
- le système de maintien comporte une pièce de maintien, qui est fixée ou apte à être fixée à la partie extrême avant du guide souple, et qui est adaptée pour pouvoir être appliquée sur le corps d'un patient de manière à maintenir temporairement la partie extrême avant du guide par rapport au corps d'un patient, à proximité de la zone d'insertion de l'élément filaire de délivrance de doses de traitement.
- le système de maintien comporte des moyens de fixation qui permettent de fixer temporairement la pièce de maintien appliquée sur le corps d'un patient à proximité du point d'insertion de l'élément filaire de délivrance de doses de traitement, et de préférence qui comportent un adhésif apte à être collé sur la peau.
- la pièce de positionnement et guidage, l'élément filaire de délivrance de doses de traitement, le cas échéant le guide souple, et le cas échéant le support de stockage, sont stériles et placés dans un emballage hermétique.

L'invention a également pour objet un dispositif de traitement endoveineux comportant l'un ou l'autre des ensembles susvisés et un système d'entraînement, de préférence motorisé, qui comprend des moyens d'entraînement, le dispositif comprenant en outre des deuxièmes moyens d'assemblage mécanique adaptés pour coopérer avec les premiers moyens d'assemblage mécanique de la pièce de positionnement et guidage, de manière à permettre ledit montage amovible de la pièce de positionnement et guidage par rapport au système d'entraînement, de telle sorte que ladite première portion de l'élément filaire de délivrance de doses de traitement est positionnée de manière à pouvoir être en prise avec des moyens d'entraînement du système d'entraînement et que les moyens d'entraînement du système d'entraînement permettent l'entraînement de l'élément filaire de délivrance de doses de traitement dans le sens de sa longueur au moins dans une première direction, avec de préférence un déplacement de l'élément filaire de délivrance de doses de traitement dans le sens de sa longueur par rapport auxdits moyens d'entraînement.

Plus particulièrement, le dispositif de l'invention peut comporter les caractéristiques additionnelles et optionnelles suivantes, prises isolément, ou en combinaison les unes avec les autres :
- les deuxièmes moyens d'assemblage mécanique sont solidaires ou font partie intégrante du système d'entraînement.
- les moyens d'entraînement du système d'entraînement comportent au moins une paire de galets d'entraînement et les deuxièmes moyens d'assemblage mécanique sont adaptés pour coopérer avec les premiers moyens d'assemblage mécanique de la pièce de positionnement et guidage, de manière à permettre le positionnement de la première portion de l'élément filaire de délivrance de doses de traitement entre les galets d'entraînement.
- les moyens d'entraînement du système d'entraînement permettent un entraînement par friction de l'élément filaire (1) de délivrance de doses de traitement.
- le dispositif de traitement endoveineux comporte en outre une source de doses de traitement apte à être connectée à l'extrémité arrière de l'élément filaire de délivrance de doses de traitement.
- le système d'entraînement et la source de doses de traitement font partie intégrante d'un même ensemble monolithique.
- l'élément filaire de délivrance de doses de traitement est une fibre optique et la source de doses de traitement est une source de rayonnement électromagnétique.

L'invention a également pour objet une utilisation de l'ensemble susvisé ou du dispositif de traitement endoveineux susvisé pour traiter une veine, et notamment pour traiter une veine au moyen d'un rayonnement électromagnétique.

L'invention a également pour objet un procédé de préparation d'un dispositif de traitement endoveineux susvisé ou un procédé de traitement endoveineux au moyen du dispositif de traitement endoveineux susvisé, au cours duquel on monte de manière amovible, la pièce de positionnement et guidage par rapport au système d'entraînement, et de préférence sur le système d'entraînement, de telle sorte qu'une première portion de l'élément filaire de délivrance de doses de traitement est positionnée de manière à pouvoir être en prise avec les moyens d'entraînement du système d'entraînement et peut être entraînée au moins dans une première direction d'entraînement.

### Brève description des dessins

Les caractéristiques et avantages de l'invention apparaîtront plus clairement à la lecture de la description détaillée ci-après d'une variante particulière de réalisation de l'invention, laquelle variante particulière de réalisation est décrite à titre d'exemple non limitatif et non exhaustif de l'invention, et en référence aux dessins annexés sur lesquels :
- la figure 1 est une vue schématique d'ensemble montrant un exemple de mise en oeuvre d'un dispositif de traitement endoveineux de l'invention, de type laser endoveineux, pour traiter par laser une veine d'une jambe,
- la figure 2 est une vue isométrique d'une variante particulière d'un ensemble de traitement endoveineux de l'invention, conditionné dans sa configuration de transport et/ou stockage,
- la figure 3 est une vue isométrique de l'ensemble de la figure 2, une fois le support de stockage séparé de la pièce de positionnement et guidage, et la pièce de positionnement et guidage étant montée sur un système d'entraînement,
- la figure 4 est une vue isométrique du support de stockage (sans la fibre optique et sans la gaine) de l'ensemble de la figure 2,
- la figure 5 représente un exemple de système d'entraînement,
- la figure 6 est une vue isométrique de l'ensemble de la figure 3 en cours de montage de la pièce de positionnement et guidage sur le système d'entraînement de la figure 5,
- la figure 7 est une vue isométrique de l'ensemble de la figure 3, une fois la pièce de positionnement et guidage montée sur le système d'entraînement,
- la figure 8 est une vue de détail montrant la fixation de la partie arrière de la gaine sur la pièce de positionnement et guidage,
- la figure 9 est une vue en coupe transversale montrant l'indexation des pattes d'assemblage de la pièce de positionnement et guidage par rapport au système d'entraînement,
- la figure 10 est une vue isométrique d'un exemple de système de maintien de la gaine mis en place et fixé sur une partie de corps humain, avec la fibre optique insérée à travers la peau,

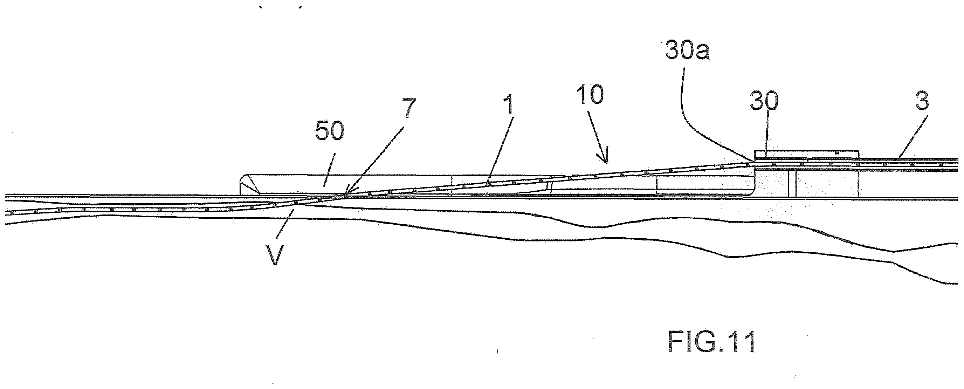

- la figure 11 est une vue en coupe longitudinale de la figure 10, montrant notamment la veine et la fibre optique insérée dans la veine,
- La figure 12 est une vue isométrique illustrant une étape d'insertion de la partie extrême avant de la fibre optique dans une veine, au moyen d'un cathéter d'introduction,
- La figure 13 est une vue isométrique montrant le cathéter d'introduction de la figure 12, retiré de la veine, une fois la partie extrême avant de la fibre optique introduite dans une veine.

### Description détaillée

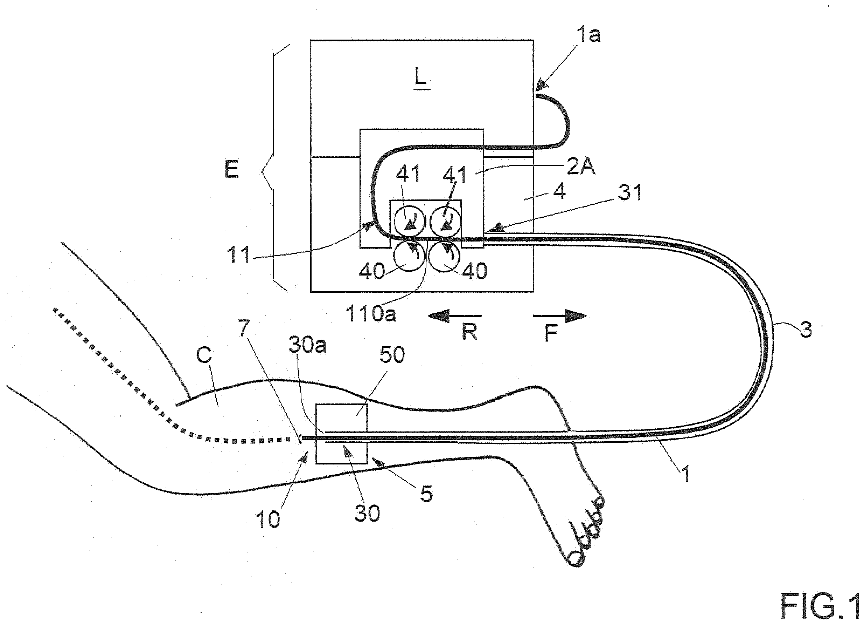

On a représenté de manière schématique sur la figure 1 un dispositif de traitement endoveineux à retrait contrôlé, conforme à l'invention, en cours d'utilisation pour le traitement d'une veine.

Ce dispositif de traitement endoveineux à retrait contrôlé comporte :
- un élément filaire souple 1 pour la délivrance de doses de traitement dans la veine, lequel élément filaire souple 1 est une fibre optique dans l'exemple particulier décrit en détail ci-après,
- un système d'entraînement motorisé 4 qui permet de tirer de manière contrôlée et vers l'arrière (direction R) sur la fibre optique 1,
- une pièce de positionnement et guidage 2A qui sera décrite en détail ci-après, et qui permet un positionnement rapide et fiable et un guidage en translation de la fibre optique 1 par rapport aux moyens d'entraînement du système d'entrainement motorisé 4,
- une source de rayonnement électromagnétique L, de type source laser, qui est couplée à l'extrémité arrière 1a de la fibre optique 1,
- une gaine de guidage souple 3, qui entoure et guide la fibre optique 1 sur une portion avant de sa longueur, la fibre optique 1 pouvant coulisser longitudinalement par rapport à la gaine 3.

En référence à la figure 1, la gaine de guidage souple 3 comporte une partie extrême arrière 31 et à l'opposé une partie extrême avant 30, qui se termine par une ouverture avant 30a permettant le passage de la fibre optique 1. La fibre optique 1 est enfilée dans la gaine de guidage 3, de telle sorte que la gaine de guidage 3 entoure et guide la fibre optique 1 sur une portion de sa longueur, avec une partie arrière 11 de la fibre optique 1 et à l'opposé une partie avant 10 de la fibre optique 1 positionnées en dehors de la gaine de guidage 3. L'extrémité avant de la fibre optique 1 qui permet l'émission du rayonnement électromagnétique dans la veine est ainsi positionnée en dehors de la gaine de guidage 3.

La partie extrême arrière 31 de la gaine souple est solidaire de la pièce de positionnement et guidage 2A, de manière à bloquer axialement cette partie arrière 31 de la gaine de guidage 3, par rapport à la fibre optique 1, au moins dans la direction de retrait R de la fibre optique 1, et de préférence également dans la direction opposée d'avancement F de la fibre optique 1, la fibre optique 1 pouvant coulisser longitudinalement par rapport à la gaine de guidage 3. Le système d'entrainement motorisé 4 permet ainsi de tirer vers l'arrière (flèche R) la fibre optique 1 en la faisant coulisser par rapport à la gaine 3.

La gaine 3 doit permettre un coulissement de la fibre optique 1 avec de préférence un minimum de frottement et doit de préférence être biocompatible. Le diamètre intérieur de la gaine 3 doit être également ajusté par rapport au diamètre extérieur de la fibre optique 1, afin de limiter les déplacements radiaux de la fibre optique 1 dans la gaine 3 et permettre une transmission efficace des mouvements longitudinaux. Si l'écart entre le diamètre intérieur de la gaine 3 et le diamètre extérieur de la fibre optique 1, est trop important, on peut occasionner un temps de latence préjudiciable entre le moment où on active le moteur du système d'entraînement 4 et le moment où on constate le retrait effectif de la fibre par rapport à la gaine. A titre d'exemples non limitatifs et non exhaustifs, avec une fibre optique 1 présentant un diamètre extérieur de 900µm on utilisera une gaine 3 présentant par exemple un diamètre intérieur de 1000µm, et avec une fibre optique 1 présentant un diamètre extérieur de 600µm, on utilisera une gaine 3 présentant par exemple un diamètre intérieur de 700µm.

Différents matériaux peuvent être utilisés pour la gaine 3, parmi lesquels et manière non limitative et non exhaustive les matériaux suivants : silicone, polyuréthane, PTFE, PET, ETFE, latex, élastomère thermoplastique.

Dans la variante particulière de réalisation de la figure 1, mais de manière non limitative de l'invention, le dispositif comporte également un système de maintien 5 qui permet de fixer temporairement la partie extrême avant 30 de la gaine de guidage 3 sur le corps C d'un patient (en l'occurrence sur la figure 1 et de manière non limitative sur une jambe) à proximité du point d'insertion 7 de la fibre optique dans le corps C.

Il convient de noter que dans le cadre de l'invention, la gaine de guidage souple 3 et/ou la pièce de maintien 5 sont facultatives et pourraient ne pas être mises en oeuvre dans une autre variante de réalisation de l'invention.

Le système d'entrainement 4 comporte deux paires de galets d'entraînement rotatifs 40, 41, entre lesquels une première portion 110a rectiligne de la fibre optique 1 est positionnée et guidée par la pièce 2A. Les galets 40 sont par exemple des galets entraîneurs motorisés et les galets 41 sont par exemple des galets montés libre en rotation. Ces galets d'entraînement rotatifs 40, 41 permettent d'entraîner par friction la fibre optique 1 vers l'arrière (direction R) à une vitesse contrôlée qui dépend de la vitesse de rotation des galets 40, 41 lors de l'opération de retrait contrôlé de la fibre optique par rapport à la veine à traiter. Dans cette variante particulière de réalisation, lorsque la fibre optique 1 est entraînée par friction par les galets rotatifs 40, 41, elle se déplace dans le sens de sa longueur par rapport à ces galets.

Dans une autre variante de réalisation, le système d'entraînement 4 peut comporter uniquement une paire de galets d'entraînement rotatifs 40, 41. Plus généralement, les galets d'entraînement rotatifs 40, 41 peuvent être remplacés par tout moyen équivalent remplissant la fonction d'entraînement de la fibre optique dans le sens de sa longueur avec de préférence un déplacement de la fibre optique dans le sens de sa longueur par rapport auxdits moyens d'entraînement. Cet entraînement de la fibre optique ne s'opère pas nécessairement par friction, mais d'une manière plus générale à l'aide de tout moyen d'entraînement pouvant être en prise avec la fibre optique. Les moyens d'entraînement peuvent par exemple comporter une pince, qui est mobile en translation aller-retour entre deux positions extrêmes, et qui est commandée pour saisir la première portion accessible 110a de la fibre optique 1, lors du déplacement en translation de la pince d'une première position extrême à l'autre, et pour lâcher et ne plus être en prise avec la fibre optique lors du déplacement retour de la pince en sens inverse. Dans la variante de réalisation de la figure 1, le système d'entraînement 4 pour le retrait contrôlé de la fibre optique 1 et la source de rayonnement électromagnétique L font avantageusement partie intégrante d'un même ensemble monolithique E. Dans une autre variante de réalisation, le système d'entraînement 4 peut toutefois être séparé et distant de la source de rayonnement électromagnétique L.

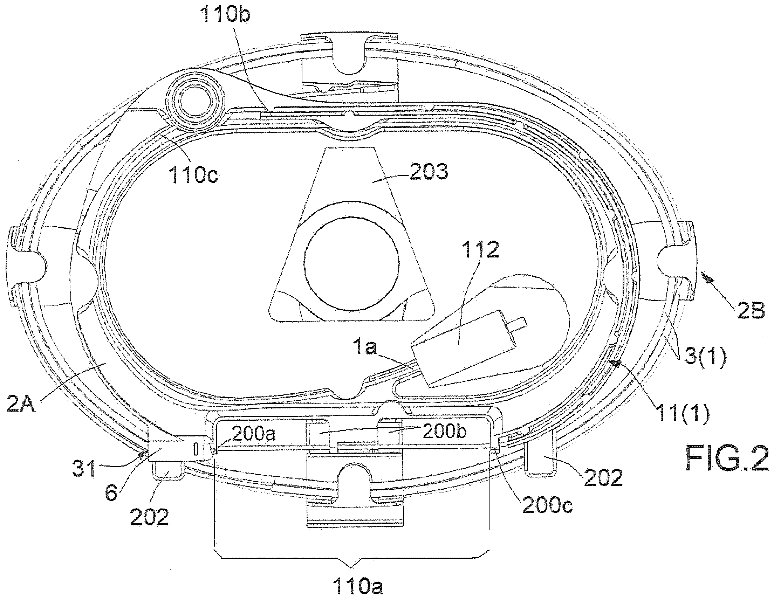

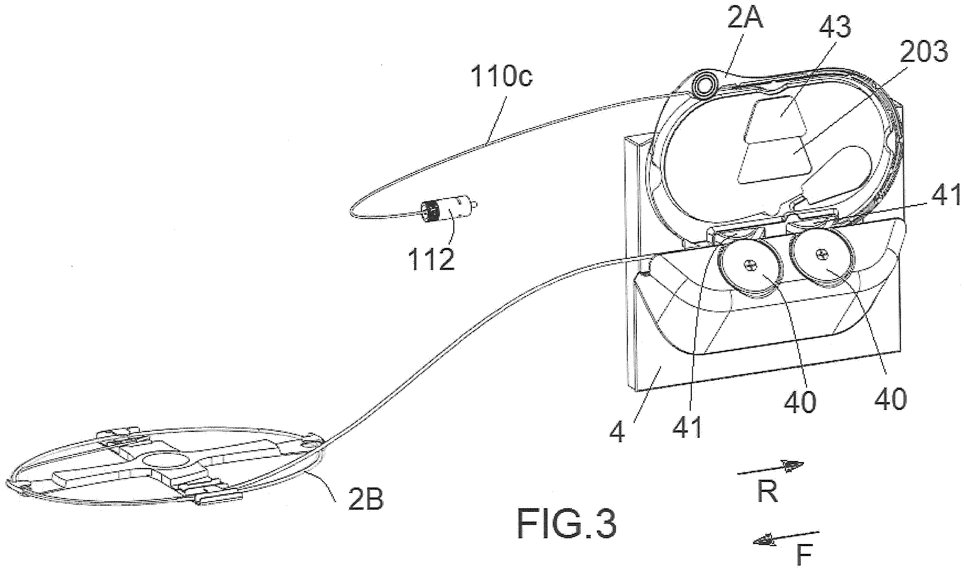

En référence aux figures 2 et 3, dans cette variante particulière de réalisation, le dispositif endoveineux comporte également un support de stockage 2B sur lequel sont enroulés tout ou partie de la portion de la fibre optique 1 qui s'étend depuis l'extrémité avant la fibre optique 1 jusqu'à ladite première portion 110a de la fibre optique 1 et tout ou partie de la gaine souple 3 enfilée sur la fibre optique 1. Ce support de stockage 2B permet de faciliter le transport, le stockage et la manipulation de la fibre optique 1.

Dans la variante particulière de réalisation illustrée sur les figures 2 à 4, ce support de stockage 2B et la pièce de positionnement et guidage 2A sont séparés, et peuvent plus particulièrement être superposés à plat l'un sur l'autre de manière à limiter leur encombrement en cours de transport. Plus particulièrement, ce support de stockage 2B et la pièce de positionnement et guidage 2A peuvent dans une variante de réalisation être assemblés temporairement par exemple par clipsage, et être facilement séparables.

L'ensemble de la figure 2 est de préférence conditionné pour son transport dans un emballage hermétique (non représenté), par exemple un sachet ou un emballage coque de type « blister », après avoir été préalablement stérilisé. Cet ensemble de la figure 2 est retiré de son emballage par le praticien dans le champ opératoire stérile, avant de procéder au traitement endoveineux.

Cet ensemble de la figure 2 peut avantageusement, mais non nécessairement être jetable et à usage unique.

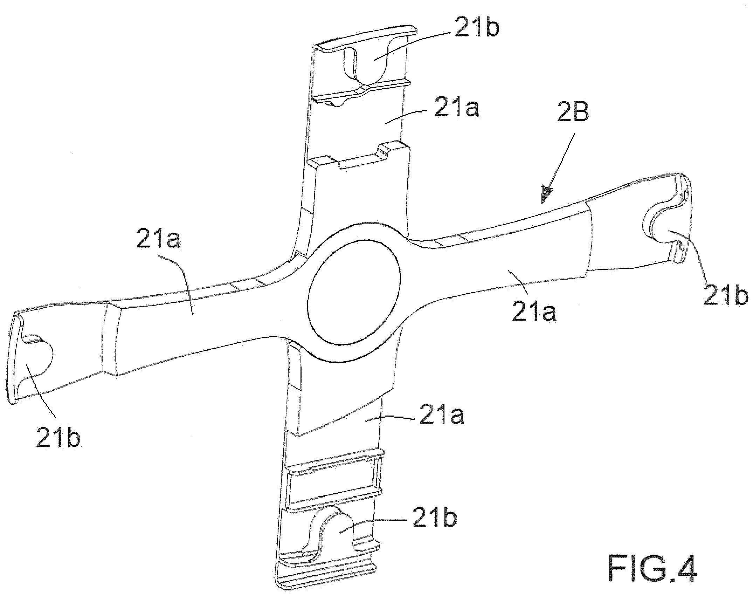

Dans la variante particulière de réalisation illustrée sur les figures, le support de stockage 2B forme (figure 4) une pièce rigide en forme de croix à quatre branches 21a est par exemple une pièce monobloc en plastique. La gaine 3 et la fibre optique 1 sont enroulées sur cette croix (figure 2), en étant insérées et bloquées entre des clips élastiques 21b à chaque extrémité des bras 21a de la croix. Une fois le support de stockage 2B séparé de la pièce de positionnement et guidage 2A (figure 3), la gaine 3 et la portion de la fibre optique 1 enroulées sur ce support de stockage 2B peuvent être facilement retirées manuellement du support de stockage 2B par un opérateur et être déroulées pour leur utilisation.

Dans le cadre de l'invention, toute autre forme et/ou tout type de matériau pour réaliser cette pièce à fonction de support de stockage 2B sont envisageables.

En outre dans le cadre de l'invention, ce support de stockage 2B est facultatif, l'ensemble endoveineux pouvant comporter uniquement la pièce 2A de positionnement et guidage et l'élément filaire 1 de délivrance de doses de traitement, enfilé le cas échéant sur un guide souple 3.

Dans la variante particulière de réalisation des figures annexées, la pièce de positionnement et guidage 2A est une pièce plate rigide monobloc, par exemple une pièce moulée en plastique. Toute autre forme et/ou tout autre matériau pour réaliser cette pièce de positionnement et guidage 2A sont envisageables.

La pièce de positionnement et guidage 2A n'est pas nécessairement monobloc et peut dans une autre variante être constituée par un assemblage rigide et monolithique de plusieurs éléments entre eux.

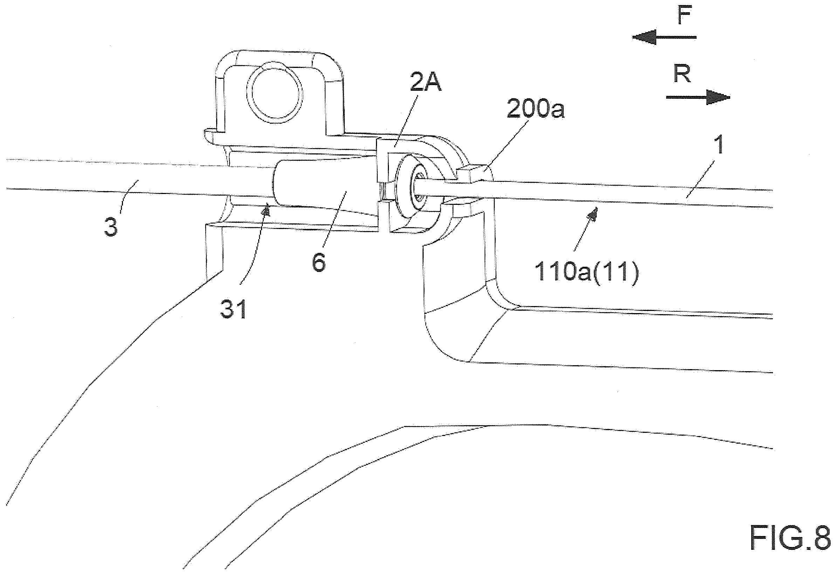

La gaine 3 comporte à son extrémité arrière 31 un connecteur 6 (figures 8) qui est adapté pour être emboîté sur la pièce de positionnement et guidage 2A, de manière à rendre l'extrémité arrière 31 de la gaine 3 solidaire de la pièce de positionnement et guidage 2A, en obtenant un blocage axial de la partie arrière de la gaine 3 au moins dans la première direction de retrait R vers l'arrière de l'élément filaire 1 de délivrance de doses de traitement, et de préférence également dans la direction F (vers l'avant) opposée à la direction de retrait R de la fibre optique 1. Ce connecteur 6 peut également, mais non nécessairement, être fixé de manière définitive à la pièce 2A, par exemple par collage.

La portion arrière 11 de la fibre optique 1 qui dépasse de l'extrémité arrière de la gaine 3 est solidaire de la pièce de positionnement et guidage 2a, de manière à permettre la manipulation de la fibre optique 1 au moyen de cette pièce 2A de positionnement et guidage, et la fibre optique 1 peut coulisser dans le sens de sa longueur par rapport à ladite pièce de positionnement et guidage 2A, en étant en partie guidée par cette pièce 2A, tel que cela va à présent être détaillé.

Plus particulièrement, en référence notamment à la figure 2, la portion arrière 11 de la fibre optique 1 comporte une première portion 110a de fibre optique 1, qui s'étend vers l'arrière de la fibre optique 1 à partir de l'extrémité arrière de la gaine 3 pourvue du connecteur 6, et qui est de préférence rectiligne.

La pièce de positionnement et guidage 2A comporte des premiers moyens de guidage sous la forme de trois éléments de guidage 200a, 200b 200c, qui sont alignés et dans lesquels est enfilée cette première portion 110a de fibre optique 1. Ces éléments de guidage 200a, 200b, 200c permettent de solidariser la fibre optique 1 à la pièce 2A en guidant en translation la fibre optique 1 dans cette première portion 110a rectiligne, lors du coulissement de la fibre optique 1 dans le sens de sa longueur par rapport à la pièce 2A.

Dans une autre variante, la pièce de positionnement et guidage 2A peut ne comporter qu'un seul élément de guidage 200a, 200b ou 200c ou deux éléments de guidage espacés ou plus de trois éléments de guidage espacés,

Cette première portion 110a de fibre optique 1 est de préférence accessible de manière à permettre son positionnement de manière à pouvoir être en prise avec les moyens d'entraînement du système d'entraînement 4, c'est-à-dire dans la variante particulière illustrée sur les figures de manière à permettre son positionnement entre les rouleaux ou galets d'entraînement 40, 41.

De manière usuelle, le système d'entraînement est équipé d'un moyen d'embrayage/débrayage des rouleaux ou galets d'entraînement 40, 41. Lors du montage de la pièce 2A sur le système d'entraînement, les rouleaux ou galets d'entraînement 40, 41 sont débrayés de telle sorte qu'ils sont libres en rotation; la première portion 110a de fibre optique 1 est positionnée entre les rouleaux ou galets d'entraînement 40, 41, sans être en prise avec ses rouleaux ou galets d'entraînement 40, 41, ce qui permet de pouvoir coulisser la fibre optique à la main. Ensuite, l'opérateur embraye au moins l'un des deux rouleaux ou galets d'entraînement 40, 41 pour son entrainement motorisé, de manière à ce que la première portion 110a de fibre optique 1 soit en prise avec les rouleaux ou galets d'entraînement 40, 41 et puisse être entraînée par friction par les rouleaux ou galets d'entraînement 40, 41,

Dans une autre variante, le système d'entraînement peut être équipé de manière usuelle d'un moyen d'ajustement de l'entre-axe entre les rouleaux ou galets d'entraînement 40, 41, qui permet à un opérateur de déplacer les rouleaux ou galets d'entraînement 40, 41 entre une position écartée et une position rapprochée dans laquelle ils sont aptes à être en prise avec la fibre optique 1 et à l'entraîner par friction. Lors du montage de la pièce 2A sur le système d'entraînement, les rouleaux ou galets d'entraînement 40, 41 sont en position écartée et la première portion 110a de fibre optique 1 est positionnée entre les rouleaux ou galets d'entraînement 40, 41, sans être en prise avec ses rouleaux ou galets d'entraînement 40, 41, ce qui permet de pouvoir coulisser la fibre optique à la main. Ensuite, l'opérateur commande le rapprochement des rouleaux ou galets d'entraînement 40, 41, de manière à ce que la première portion 110a de fibre optique 1 soit en prise avec les rouleaux ou galets d'entraînement 40, 41 et puisse être entraînée par friction par les rouleaux ou galets d'entraînement 40, 41,

Dans une autre variante, cette première portion 110a de fibre optique 1 pourrait être protégée temporairement par un moyen de protection, qui peut être séparé de la pièce de positionnement et de guidage 2A ou qui peut être mobile par rapport à la pièce de positionnent et de guidage 2A de manière rendre accessible cette première portion 110a de fibre optique 1 avant le montage de la pièce de positionnement et de guidage 2A par rapport aux moyens d'entraînement du système d'entraînement 4 ou une fois la pièce de positionnement et de guidage 2A montée par rapport aux moyens d'entraînement du système d'entraînement 4.

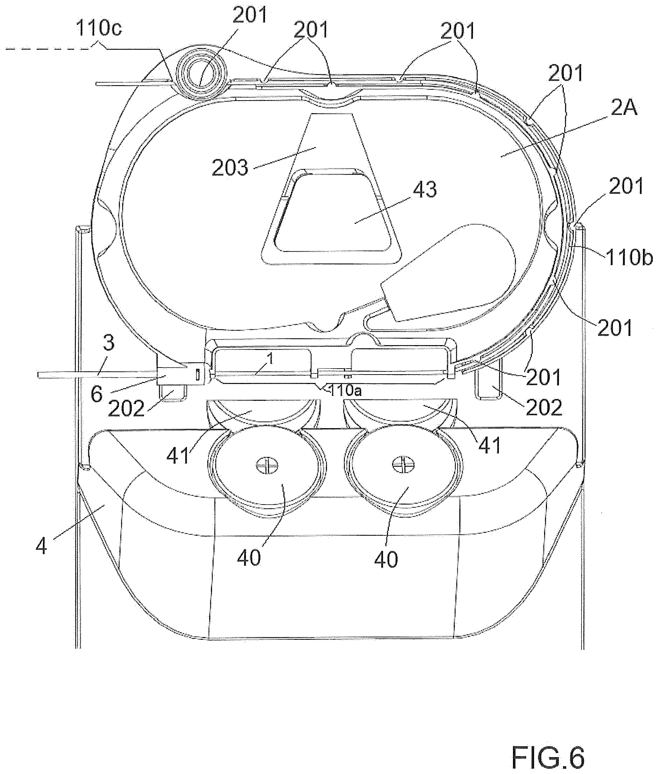

Dans cette variante, la première portion 110a de fibre optique 1 se prolonge vers l'arrière par une deuxième portion 110b (figure 6), et la pièce 2A comporte des deuxièmes moyens de guidage 201, qui permettent de solidariser la fibre optique 1 à la pièce 2A et de guider en translation la fibre optique 1 dans cette deuxième portion 110b, lors du coulissement de la fibre optique 1 dans le sens de sa longueur par rapport à la pièce 2A.

Plus particulièrement, dans cette variante de réalisation cette deuxième portion 110b est courbe, les deuxièmes moyens de guidage guidant en translation cette deuxième portion 110b de la fibre optique 1 en lui faisant avantageusement effectuer un demi-tour.

La deuxième portion 110b de fibre optique 1 se prolonge jusqu'à l'extrémité arrière 1a de la fibre optique 1 par une troisième portion 110c (figures 2 et 6). A cette extrémité arrière de la fibre optique 1 est fixé un coupleur optique 112 pour le couplage de la fibre optique à la source laser L. Dans cette variante de réalisation, cette troisième portion 110c de la fibre optique 1 et le coupleur optique 112 sont solidaires temporairement de la pièce 2A, en étant emboîtés dans la pièce 2A et peuvent être facilement retirés manuellement de la pièce 2A par un opérateur (figure 3) et être déroulés afin de coupler la fibre optique 1 à la source laser L.

Dans une autre variante de réalisation, cette troisième portion 110c de la fibre optique 1 et le coupleur optique 112 peuvent être tout le temps désolidarisés de la pièce 2A.

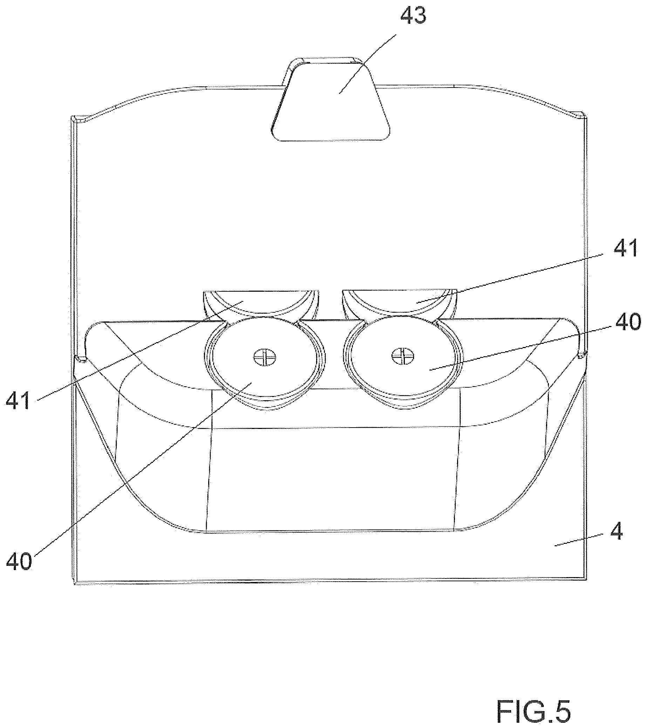

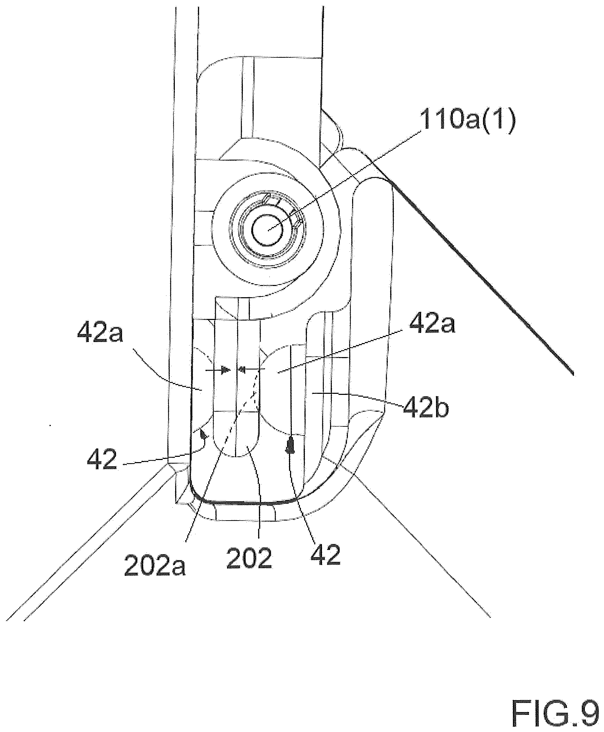

En référence à la figure 2, la pièce de positionnement et guidage 2A comporte des premiers moyens d'assemblage mécanique 202, 203 aptes à coopérer avec des deuxièmes moyens d'assemblage mécanique 42,43, qui dans cette variante font partie intégrante du système d'entraînement 4 (figures 5 et 9). Ces premiers 202, 203 et deuxièmes 42, 43 moyens d'assemblage mécanique permettent un montage amovible, rapide et sans outil, de la pièce de positionnement et guidage 2A par rapport au système d'entraînement 4, et plus particulièrement dans cette variante un montage amovible, rapide et sans outil, de la pièce 2A sur le système d'entraînement 4.

Une fois la pièce de positionnement et guidage 2A montée par rapport au système d'entraînement 4, ladite première portion 110a de la fibre optique 1 est positionnée entre les galets d'entraînement 40, 41 du système d'entraînement 4 pour l'entraînement par friction vers l'arrière (flèche R) de la fibre optique 1 par les galets d'entraînement 40, 41.

Dans cette variante particulière de réalisation et de manière non limitative de l'invention, en référence aux figures 2 et 9, les premiers moyens d'assemblage mécanique comportent un évidement 203 dans la pièce 2A et deux pattes d'assemblage 202 qui font partie intégrante de la pièce 2A et qui comportent au moins sur une face un logement hémisphérique 202a (figure 9). En référence aux figures 5, 6, 7 et 9, dans cette variante les deuxièmes moyens d'assemblage mécanique font partie intégrante du système d"entraînement 4 et comportent un élément d'accrochage 43, qui est adapté pour coopérer avec l'évidement 203 dans la pièce 2A et deux éléments d'indexage 42 pour chaque patte d'assemblage 202 (figure 9), qui sont positionnés de part et d'autre de la patte d'assemblage 202, lorsque la pièce 2A est montée sur le système d'entraînement 4.

En référence à la figure 9, chaque élément d'indexage 42 comporte une bille 42a qui est repoussée élastiquement en direction de la patte d'assemblage 202 dans la position de blocage de la figure 9, par un ressort (non visible) logé dans un corps cylindrique 42b. Lorsque la pièce 2A est montée sur le système d'entraînement 4, l'une des deux billes 42a (bille de droite sur la figure 9) est repoussée élastiquement de manière à venir se loger en partie dans le logement hémisphérique 202a de la patte d'assemblage 202 et l'autre bille 42a (bille de gauche sur la figure 9) est repoussée élastiquement de manière à venir en appui contre la patte d'assemblage 202.

Initialement, en référence à figure 2, la plus grande portion de la fibre optique 1 entourée par la gaine 3 est conditionnée en étant enroulée sur le support de stockage 2B. Le connecteur 6 à l'extrémité arrière 31 de la gaine 3 est solidaire de la pièce de positionnement et guidage 2A, tel que précédemment décrit. La partie arrière 11 de la fibre optique 1 dépourvue de gaine 3 est solidaire de la pièce de positionnement et guidage 2A, tel que précédemment décrit ; le support de stockage 2B est superposé avec la pièce de positionnement et guidage 2A. L'ensemble est ainsi peu encombrant, et peut être facilement manipulé et/ou stocké et/ou transporté.

Pour positionner la fibre optique 1 de cet ensemble par rapport au système d'entraînement 4, on procède de la manière suivante.

On sépare le cas échéant le support de stockage 2B et la pièce de positionnement et guidage 2A.

On retire de la pièce de positionnement et guidage 2A, la troisième portion 110c de la fibre optique portant à son extrémité le coupleur optique 112 (figure 3).

On monte la pièce de positionnement et guidage 2A avec la fibre optique 1 sur le système d'entraînement 4, ce qui permet de positionner facilement, rapidement et avec précision, la première portion rectiligne 110a de la fibre optique entre les galets d'entraînement 40, 41.

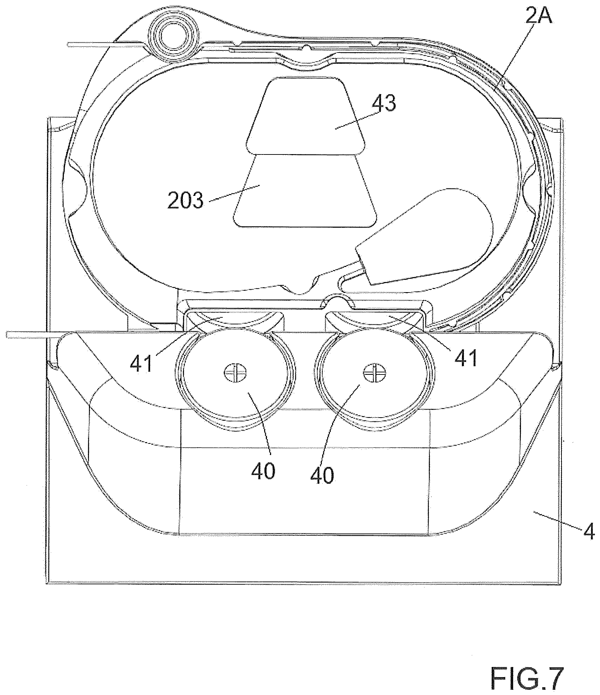

Dans cette variante particulière de réalisation, ce montage est réalisé en deux phases. Dans une première phase (figure 6) on positionne la pièce 2A par rapport au système d'entraînement 4, en alignant verticalement et horizontalement l'évidement 203 de la pièce 2A par rapport à l'élément d'accrochage 43, tel qu'illustré sur la figure 6. Dans une deuxième phase (figure 7), on descend verticalement la pièce 2A par rapport au système d'entraînement 4, de manière à insérer le bord supérieur de l'évidement 203 dans l'élément d'accrochage 43.

Lors de ce mouvement de descente, les pattes d'assemblage 202 se positionnent entre leurs éléments d'indexage 42. Cette descente de la pièce 2A est effectuée jusque dans la position d'indexage de la figure 9.

Ce montage amovible de la pièce 2A permet à un opérateur de positionner rapidement et de manière fiable la première portion 110a de la fibre optique 1 entre les galets d'entraînement 40, 41. Lorsque ces galets 40, 42 entraînent la fibre optique 1 en tirant sur cette première portion 110a, la fibre optique 1 coulisse par rapport à la gaine 3 en étant guidée par les moyens de guidage 200a, 200b, 200c et 201 de la pièce 2A.

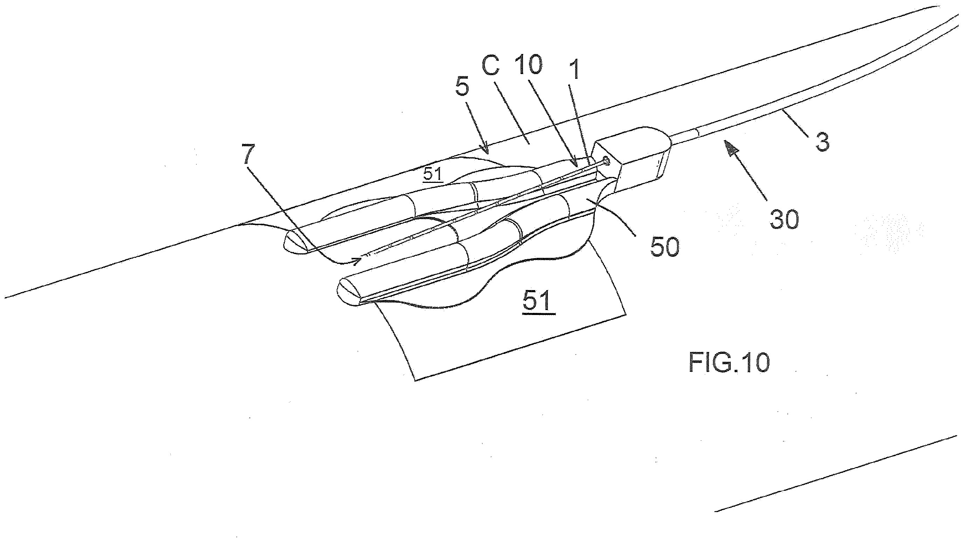

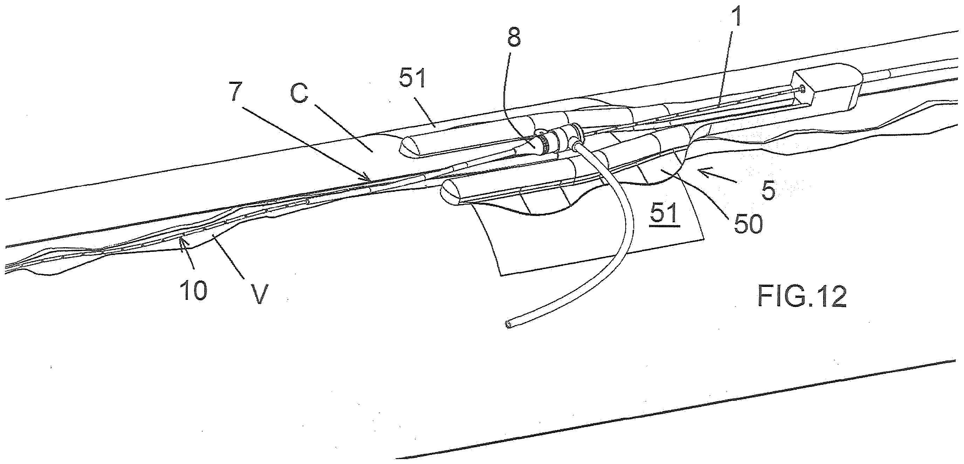

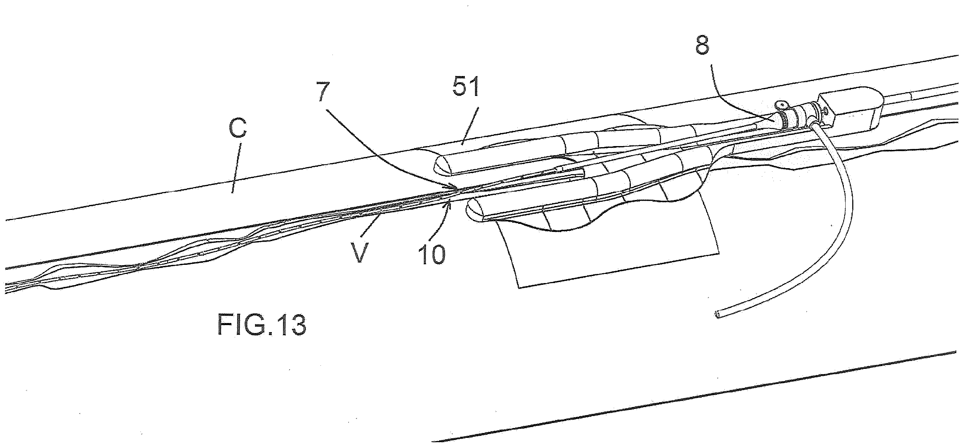

Une fois le montage de la pièce 2A effectué, il suffit ensuite de fixer la partie avant 30 de gaine 3 à la pièce de maintien 50 du système de maintien 5 (figure 10) et le dispositif est alors prêt à être utilisé pour réaliser un traitement endoveineux, par exemple et de manière non limitative de l'invention de la manière suivante.
(a) On fixe la partie extrême avant 30 de la gaine par rapport au corps C, en fixant la pièce de maintien 50 sur le corps humain C à proximité du point d'insertion 7 de la fibre optique 1, par exemple au moyen d'un adhésif 51 (figure 10).
(b) On enfonce de manière usuelle, à travers la peau et dans la veine V à traiter, une aiguille creuse, communément appelée aiguille de ponction, dont la pointe est localisée par ultrasons au moyen d'une sonde échographique. Le point d'insertion de cette aiguille correspond au point d'insertion 7 susvisé.
(c) On insère un fil-guide dans cette aiguille creuse jusque dans la veine à traiter, puis on retire l'aiguille.
(d) On enfile un cathéter d'introduction 8 sur le fil-guide jusqu'à l'entrée de la veine V et on retire le fil-guide (figure 12).
(e) Une fois le cathéter d'introduction 8 mis en place (figure 12), on insère la partie extrême avant 10 de la fibre optique 1, qui dépasse en dehors de la partie extrême avant 30 de la gaine 3, dans le cathéter d'introduction 8 et on fait coulisser la fibre optique 1 vers l'avant par rapport à la gaine 3, jusqu'à ce que l'extrémité de la partie extrême avant 10 de fibre optique 1 pénètre longitudinalement dans la veine V et progresse longitudinalement dans la veine V jusqu'à la zone à traiter la plus éloignée du point d'insertion 7. Pendant cette opération, le moteur d'entraînement des galets du système d'entraînement 4 est débrayé.
(f) Une fois la fibre optique 1 introduite et positionnée dans la veine V, on retire le cathéter 8 de la veine V en le faisant coulisser vers l'arrière sur la fibre optique 1 (figure 13). Eventuellement on retire le cathéter 8 de la fibre optique 1, par exemple en le fendant en deux dans le cas d'un cathéter déchirable. Dans une autre variante, le cathéter peut être retiré à l'issue de la procédure de traitement.

Le praticien peut alors pratiquer de manière usuelle le traitement endoveineux en actionnant manuellement la source laser L, afin d'émettre dans la veine un rayonnement électromagnétique dans la région de l'extrémité de la partie proximale de la fibre optique 1 et en commandant le retrait continu ou pas à pas de la fibre optique 1 au moyen du système de retrait 4 motorisé.

Grâce à la gaine de guidage 3, dont la partie extrême avant 30 est fixée temporairement au corps C, à proximité du point d'insertion 7 de la fibre optique 1, et dont la partie extrême arrière 31 est bloquée axialement par rapport à la fibre optique 1 au moyen du connecteur 6, le traitement endoveineux peut avantageusement être réalisé sans que la fibre optique 1 ne soit tendue et en réduisant les risques de déplacements accidentels de la fibre optique par rapport à la veine en cours de traitement.

Une fois le traitement laser terminé, la fibre optique 1 est totalement retirée de la veine et le système de maintien 5 est désolidarisé du corps humain. Le praticien peut alors déconnecter la fibre optique 1 de la source laser L, et retirer la pièce 2A avec la fibre optique 1 du système d'entraînement 4.

Dans la variante de réalisation de la figure 1, la partie arrière 11 de la fibre optique 1 est manière avantageuse guidée et renvoyée vers l'avant par les deuxièmes moyens de guidage 201 de la pièce 2A, grâce au demi-tour effectué par la deuxième portion arrière 110b de la fibre 1. Ainsi, lors du retrait de la fibre optique 1 vers l'arrière (flèche R), la fibre optique1 étant maintenue et guidée par les deuxièmes moyens de guidage 201, on évite les risques d'accrochage accidentel dans la fibre optique par une personne ou un objet.

Plus généralement, les deuxièmes moyens de guidage 201 peuvent être conçus pour guider en translation cette deuxième portion 110b de l'élément filaire 1 de délivrance de doses de traitement en lui faisant effectuer au moins un quart de tour.

Dans une autre variante de réalisation, la deuxième portion 101b de la fibre optique 1 qui est guidée de manière à effectuer au moins un quart de tour n'est pas nécessairement située dans le prolongement arrière de la première portion 101a de la fibre optique 1, mais peut être positionnée dans le prolongement avant de la première portion 101a de la fibre optique 1. En outre les deuxièmes moyens de guidage 202 ne font pas nécessairement partie intégrante de la pièce 2A, mais pourraient être des moyens de guidage distincts et séparés de cette pièce 2A.

De préférence, les deuxièmes moyens d'assemblage mécanique 42,43 sont solidaires ou font partie intégrante du système d'entraînement 4. Néanmoins, dans une autre variante de réalisation, les deuxièmes moyens d'assemblage mécanique 42,43 peuvent être séparés du système d'entraînement 4 et peuvent par exemple être fixés ou faire partie intégrante d'un support de type table, sur lequel serait posé le système d'entraînement 4.

L'invention n'est pas limitée à un dispositif de traitement endoveineux par laser. Dans d'autres variantes de réalisation couvertes par l'invention, la fibre optique peut être remplacée par un élément filaire (plein ou creux) par exemple de type câble ou sonde souple ou canule souple. Le traitement n'est pas nécessairement un traitement par laser, mais peut être tout traitement par délivrance de doses de traitement dans la veine, et notamment de doses d'énergie, délivrées par exemple sous la forme d'un rayonnement électromagnétique, au moyen d'ondes sonores ou ultrasonores, d'ondes radiofréquences, ou de doses d'énergie thermique délivrées par rayonnement et/ou par contact, ou de doses d'un produit, par exemple liquide, semi-liquide ou mousseux, permettant un traitement de la veine.

Le système de retrait 4 peut d'une manière plus générale être remplacé par tout système d'entraînement permettant d'entraîner l'élément filaire 1 de délivrance de doses de traitement dans au moins une direction d'entraînement R donnée. Ce système d'entraînement 4 du dispositif n'est pas nécessairement motorisé, mais pourrait être un système d'entraînement actionné manuellement.

Dans le cadre de l'invention, la gaine de guidage 3 peut être remplacée par tout guide souple équivalent remplissant la même fonction de guidage que la gaine 3. Par exemple, et de manière non exhaustive, la gaine 3 peut être remplacée par un guide souple en forme de gouttière, et ayant par exemple une section transversale en U, ou par un guide filaire souple torsadé autour de la fibre optique 1 ou équivalent, ou par un guide souple qui est aimanté pour permettre sa solidarisation avec l'élément filaire 1 de délivrance des doses de traitement.

Le guide souple 3 n'est pas nécessairement fait d'une seule pièce mais peut comporter plusieurs éléments assemblés. Par exemple le guide 3 peut comporter une gaine de guidage souple ou équivalent à l'extrémité avant de laquelle serait fixé un cathéter d'introduction rigide, le système de maintien 5 permettant de maintenir temporairement ce cathéter d'introduction sur le corps du patient.

Le système de maintien 5 peut comporter uniquement la pièce de maintien 50 ou équivalent et peut ne pas comporter le moyen de fixation 51 ou équivalent. Dans ce cas, la pièce de maintien 50 est utilisée pour maintenir temporairement de manière manuelle la partie extrême proximale 30 du guide 3 par rapport au corps du patient à proximité du point d'insertion 7 de l'élément filaire 1 de délivrance de doses de traitement. Ce maintien temporaire de la partie extrême proximale 30 du guide 3 par rapport au corps du patient à proximité du point d'insertion 7 de l'élément filaire 1 de délivrance de doses de traitement peut se faire en positionnant la pièce de maintien 50 ou équivalent en contact avec le patient ou en tenant la pièce de maintien 50 ou équivalent dans la main et en appliquant au contact du patient la main tenant cette pièce de maintien 50 ou équivalent.

Le système de maintien peut comporter des moyens de fixation permettant de fixer temporairement la partie extrême proximale 30 du guide 3 sur le corps d'un patient, à proximité de la zone d'insertion 7 de l'élément filaire 1 de délivrance de doses de traitement, sans mise en oeuvre de la pièce de maintien 50. Par exemple, le système de maintien peut être formé d'un ou plusieurs adhésifs aptes à être appliqués directement sur la partie extrême avant 30 du guide 3 et à être collés sur le corps du patient pour fixer temporairement la partie extrême avant 30 du guide 3 par rapport au corps du patient à proximité du point d'insertion 7 de l'élément filaire 1 de délivrance de doses de traitement.

## Revendications

1. Ensemble de traitement endoveineux comportant un élément filaire (1) de délivrance de doses de traitement, qui est souple et apte à être inséré, sur une partie de sa longueur, longitudinalement dans une veine (V), et une pièce (2A) de positionnement et guidage, qui est solidaire de l'élément filaire (1) de délivrance de doses de traitement de manière à permettre la manipulation de l'élément filaire (1) de délivrance de doses de traitement au moyen de cette pièce (2A) de positionnement et guidage, ladite pièce (2A) de positionnement et guidage comportant au moins un premier moyen (200a , 200b , 200c) de guidage en translation d'une première portion (110a) de l'élément filaire (1) de délivrance de doses de traitement dans le sens de sa longueur, l'élément filaire (1) de délivrance de doses de traitement pouvant coulisser dans le sens de sa longueur par rapport audit premier moyen (200a , 200b , 200c) de guidage en translation de ladite pièce (2A) de positionnement et guidage, en étant guidée en translation par ledit premier moyen (200a , 200b , 200c) de guidage, et ladite pièce (2A) de positionnement et guidage comportant des premiers moyens d'assemblage mécanique (202, 203) permettant son montage amovible par rapport à des moyens d'entraînement d'un système d'entraînement (4), de telle sorte que ladite première portion (110a) de l'élément filaire (1) de délivrance de doses de traitement peut être positionnée et guidée au moyen de cette pièce (2A) de positionnement et guidage de manière à pouvoir être en prise avec les moyens d'entraînement du système d'entraînement (4) pour l'entraînement de l'élément filaire (1) de délivrance de doses de traitement dans le sens de sa longueur au moins dans une première direction (R).

2. Ensemble selon la revendication 1, dans lequel ladite première portion (110a) de l'élément filaire (1) de délivrance de doses de traitement est accessible ou peut être rendue accessible.

3. Ensemble selon l'une quelconque des revendications 1 ou 2, dans lequel ladite première portion (110a) de l'élément filaire (1) de délivrance de doses de traitement peut être positionnée et guidée au moyen de la pièce (2A) de positionnement et guidage de manière à pouvoir être en prise avec des moyens d'entraînement du système d'entraînement (4) pour l'entraînement de l'élément filaire (1) de délivrance de doses de traitement dans le sens de sa longueur au moins dans une première direction (R), avec un déplacement de l'élément filaire (1) de délivrance de doses de traitement dans le sens de sa longueur par rapport auxdits moyens d'entraînement.

4. Ensemble selon l'une quelconque des revendications précédentes comportant des deuxièmes moyens de guidage (201) d'une deuxième portion de l'élément filaire (1) de délivrance de doses de traitement, et dans lequel les deuxièmes moyens de guidage (201) guident en translation cette deuxième portion de l'élément filaire (1) de délivrance de doses de traitement en lui faisant effectuer au moins un quart de tour et de préférence au moins un demi-tour et/ou font partie intégrante de la pièce (2A) de positionnement et guidage .

5. Ensemble selon la revendication 4, dans lequel l'élément filaire (1) de délivrance de doses de traitement comporte une troisième portion (110c), qui s'étend jusqu'à l'extrémité arrière de l'élément filaire (1) de délivrance de doses de traitement, et qui n'est pas solidaire de la pièce (2A) de positionnement et guidage ou qui est solidaire de la pièce (2A) de positionnement et guidage mais peut être séparée de cette pièce (2A) de positionnement et guidage.

6. Ensemble selon l'une quelconque des revendications précédentes, dans lequel la pièce (2A) de positionnement et guidage est une pièce monobloc, et plus particulièrement une pièce moulée ou dans lequel la pièce (2A) de positionnement et guidage est constituée par un assemblage monolithique.

7. Ensemble selon l'une quelconque des revendications précédentes, comportant un guide souple (3) enfilé sur une portion avant de l'élément filaire (1) de délivrance de doses de traitement, ledit élément filaire (1) de délivrance de doses de traitement étant apte à coulisser longitudinalement par rapport au guide souple (3), et de préférence dans lequel l'extrémité arrière (31) du guide souple (3) est solidaire de la pièce (2A) de positionnement et guidage, de manière à bloquer axialement la partie arrière du guide souple (3) au moins dans la première direction d'entrainement (R) de l'élément filaire (1) de délivrance de doses de traitement, et de préférence également dans la direction (F) opposée à la direction d'entraînement (R) de l'élément filaire (1) de délivrance de doses de traitement.

8. Ensemble selon l'une quelconque des revendications précédentes, comportant un support de stockage (2B), sur lequel est enroulée tout ou partie de la portion avant de l'élément filaire (1) de délivrance de doses de traitement qui s'étend depuis l'extrémité avant de l'élément filaire (1) de délivrance de doses jusqu'à ladite première portion (110a) de l'élément filaire (1) de délivrance de doses de traitement, et de préférence dans lequel un guide souple (3) est enfilé sur ladite portion avant de l'élément filaire (1) de délivrance de doses de traitement, ledit élément filaire (1) de délivrance de doses de traitement étant apte à coulisser longitudinalement par rapport au guide souple (3) et le guide souple (3) et la partie de l'élément filaire (1) de délivrance de doses de traitement enfilée dans le guide souple (3) étant enroulés sur le support de stockage (2B).

9. Ensemble selon l'une quelconque des revendications précédentes, dans lequel l'élément filaire (1) de délivrance de doses de traitement est une fibre optique.

10. Ensemble selon l'une quelconque des revendications précédentes, dans lequel les premiers moyens d'assemblage mécanique (202, 203) permettent le montage amovible de la pièce (2A) de positionnement et guidage par rapport à un système d'entraînement (4) qui comprend au moins une paire de galets d'entraînement (40, 41), de telle sorte que ladite première portion (110a) de l'élément filaire (1) de délivrance de doses de traitement peut être positionnée et guidée au moyen de cette pièce (2A) de positionnement et guidage entre les galets d'entraînement (40, 41) pour l'entraînement de l'élément filaire (1) de délivrance de doses de traitement dans le sens de sa longueur au moins dans une première direction (R).

11. Ensemble selon l'une quelconque des revendications précédentes, dans lequel la pièce (2A) de positionnement et guidage, l'élément filaire de délivrance doses de traitement (1), le cas échéant le guide souple (3), et le cas échéant le support de stockage (2B), sont stériles et placés dans un emballage hermétique et/ou dans lequel les premiers moyens d'assemblage mécanique (202, 203) permettent un montage rapide et sans outil de la pièce (2A) de positionnement et guidage.

12. Dispositif de traitement endoveineux comportant un ensemble visé à l'une quelconque des revendications 1 à 9 et un système d'entraînement (4), de préférence motorisé, qui comprend des moyens d'entraînement (40 , 41), le dispositif comprenant en outre des deuxièmes moyens d'assemblage mécanique (42 43) adaptés pour coopérer avec les premiers moyens d'assemblage mécanique (202, 203) de la pièce de positionnement et guidage (2A), de manière à permettre ledit montage amovible de la pièce de positionnement et guidage (2A) par rapport au système d'entraînement (4), de telle sorte que ladite première portion (110a) de l'élément filaire (1) de délivrance de doses de traitement est positionnée de manière à pouvoir être en prise avec des moyens d'entraînement du système d'entraînement (4) et que les moyens d'entraînement du système d'entraînement (4) permettent l'entraînement de l'élément filaire (1) de délivrance de doses de traitement dans le sens de sa longueur au moins dans une première direction (R), avec de préférence un déplacement de l'élément filaire (1) de délivrance de doses de traitement dans le sens de sa longueur par rapport auxdits moyens d'entraînement.

13. Dispositif de traitement endoveineux selon la revendication 12 dans lequel les moyens d'entraînement du système d'entraînement (4) comportent au moins une paire de galets d'entraînement (40, 41) et les deuxièmes moyens d'assemblage mécanique (42,43) sont adaptés pour coopérer avec les premiers moyens d'assemblage mécanique (202, 203) de la pièce de positionnement et guidage (2A), de manière à permettre le positionnement de la première portion (110a) de l'élément filaire (1) de délivrance de doses de traitement entre les galets d'entraînement (40, 41).

14. Dispositif de traitement endoveineux selon l'une quelconque des revendications 12 à 13 dans lequel les moyens d'entraînement du système d'entraînement (4) permettent un entraînement par friction de l'élément filaire (1) de délivrance de doses de traitement.

15. Dispositif de traitement endoveineux selon l'une quelconque des revendications 12 à 14, comportant en outre une source de doses de traitement (L) apte à être connectée à l'extrémité arrière de l'élément filaire (1) de délivrance de doses de traitement, et de préférence dans lequel le système d'entraînement (4) et la source de doses de traitement (L) font partie intégrante d'un même ensemble monolithique (E).

16. Dispositif de traitement endoveineux selon la revendication 15, dans lequel l'élément filaire (1) de délivrance de doses de traitement est une fibre optique et la source de doses de traitement (L) est une source de rayonnement électromagnétique.

17. Procédé de préparation d'un dispositif de traitement endoveineux de l'une quelconque des revendications 12 à 16, au cours duquel on monte de manière amovible, la pièce (2A) de positionnement et guidage par rapport au système d'entraînement (4) de telle sorte qu'une première portion (11a) de l'élément filaire de délivrance de doses de traitement est positionnée de manière à pouvoir être en prise avec les moyens d'entraînement du système d'entraînement et peut être entraînée au moins dans une première direction d'entraînement.

## Patentansprüche

1. Endovenöse Behandlungsanordnung umfassend:
ein Leitungselement (1) zur Abgabe von Behandlungsdosen, das flexibel und auf einem Teil seiner Länge in Längsrichtung in eine Vene (V) einführbar ist, und
ein Positionierungs- und Führungsteil (2A), das mit dem Leitungselement (1) zur Abgabe von Behandlungsdosen verbunden ist, um die Bedienung des Leitungselement (1) zur Abgabe von Behandlungsdosen mittels dieses Positionierungs- und Führungsteils (2A) zu ermöglichen,
wobei das Positionierungs- und Führungsteil (2A) mindestens ein erstes Mittel (200a, 200b, 200c) zum translatorischen Führen eines ersten Abschnitts (110a) des Leitungselements (1) zur Abgabe von Behandlungsdosen in dessen Längsrichtung umfasst, wobei das Leitungselement (1) zur Abgabe von Behandlungsdosen in dessen Längsrichtung in Bezug auf das erste Mittel (200a, 200b, 200c) zum translatorischen Führen des Positionierungs- und Führungsteils (2A) gleiten kann, wobei es durch das erste Führungsmittel (200a, 200b, 200c) translatorisch geführt wird, und
wobei das Positionierungs- und Führungsteil (2A) erste mechanische Verbindungsmittel (202, 203) umfasst, die dessen lösbare Verbindung in Bezug auf Antriebsmittel eines Antriebssystems (4) erlauben, sodass der erste Abschnitt (110a) des Leitungselements (1) zur Abgabe von Behandlungsdosen mittels des Positionierungs- und Führungsteils (2A) so positioniert und geführt werden kann, dass er mit den Antriebsmitteln des Antriebssystems (4) in Verbindung sein kann, um das Leitungselement (1) zur Abgabe von Behandlungsdosen in dessen Längsrichtung zumindest in einer ersten Richtung (R) anzutreiben.

2. Anordnung nach Anspruch 1, wobei der erste Abschnitt (110a) des Leitungselements (1) zur Abgabe von Behandlungsdosen zugänglich ist oder zugänglich gemacht werden kann.

3. Anordnung nach einem der Ansprüche 1 oder 2, wobei der erste Abschnitt (110a) des Leitungselements (1) zur Abgabe von Behandlungsdosen mittels des Positionierungs- und Führungsteils (2A) so positioniert und geführt werden kann, dass er mit den Antriebsmitteln des Antriebssystems (4) in Verbindung sein kann, um das Leitungselement (1) zur Abgabe von Behandlungsdosen in dessen Längsrichtung zumindest in einer ersten Richtung (R) anzutreiben, wobei eine Verschiebung des Leitungselements (1) zur Abgabe von Behandlungsdosen in dessen Längsrichtung in Bezug auf die Antriebsmittel erfolgt.

4. Anordnung nach einem der vorhergehenden Ansprüche, mit zweiten Mitteln zum Führen (201) eines zweiten Abschnitts des Leitungselements (1) zur Abgabe von Behandlungsdosen, wobei die zweiten Führungsmittel (201) den zweiten Abschnitt des Leitungselements (1) zur Abgabe von Behandlungsdosen translatorisch führen, indem sie dieses mindestens eine Vierteldrehung und bevorzugt mindestens eine halbe Drehung ausführen lassen, und/oder einen integralen Teil des Positionierungs- und Führungsteils (2A) bilden.

5. Anordnung nach Anspruch 4, wobei das Leitungselement (1) zur Abgabe von Behandlungsdosen einen dritten Abschnitt (110c) aufweist, der bis zu dem hinteren Ende des Leitungselements (1) zur Abgabe von Behandlungsdosen (2A) verläuft, und der mit dem Positionierungs- und Führungsteil (2A) nicht fest verbunden ist, oder der mit dem Positionierungs- und Führungsteil (2A) fest verbunden ist, jedoch von dem Positionierungs- und Führungsteil (2A) abtrennbar ist.

6. Anordnung nach einem der vorhergehenden Ansprüche, wobei das Positionierungs- und Führungsteil (2A) ein einteiliges Teil ist, und insbesondere ein Formteil ist, oder wobei das Positionierungs- und Führungsteil (2A) aus einer einteiligen Anordnung aufgebaut ist.

7. Anordnung nach einem der vorhergehenden Ansprüche, mit einer flexiblen Führung (3), die auf einen vorderen Abschnitt des Leitungselements (1) zur Abgabe von Behandlungsdosen geschoben ist, wobei das Leitungselement (1) zur Abgabe von Behandlungsdosen in Bezug auf die flexible Führung (3) in Längsrichtung verschiebbar ist, und wobei bevorzugt das hintere Ende (31) der flexiblen Führung (3) fest mit dem Positionierungs- und Führungsteil (2A) verbunden ist, um den hinteren Teil der flexiblen Führung (3) zumindest in der ersten Antriebsrichtung (R) des Leitungselements (1) zur Abgabe von Behandlungsdosen axial zu blockieren, und bevorzugt auch in der Richtung (F) entgegengesetzt zur Antriebsrichtung (R) des Leitungselements (1) zur Abgabe von Behandlungsdosen.

8. Anordnung nach einem der vorhergehenden Ansprüche mit einem Speicherträger (2B), worauf alles oder ein Teil des vorderen Abschnitts des Leitungselements (1) zur Abgabe von Behandlungsdosen, der von dem vorderen Ende des Leitungselements (1) zur Abgabe von Behandlungsdosen bis zu dem ersten Abschnitt (110a) des Leitungselements (1) zur Abgabe von Behandlungsdosen verläuft, aufgewickelt ist, und wobei bevorzugt eine flexible Führung (3) auf den vorderen Abschnitt des Leitungselements (1) zur Abgabe von Behandlungsdosen geschoben ist, wobei das Leitungselement (1) zur Abgabe von Behandlungsdosen dazu geeignet ist in Bezug auf die flexible Führung (3) in Längsrichtung zu gleiten, und wobei die flexible Führung (3) und der in die flexible Führung (3) geschobene Teil des Leitungselements (1) zur Abgabe von Behandlungsdosen auf den Speicherträger (2B) aufgewickelt sind.

9. Anordnung nach einem der vorhergehenden Ansprüche, wobei das Leitungselement (1) zur Abgabe von Behandlungsdosen eine optische Faser ist.

10. Anordnung nach einem der vorhergehenden Ansprüche, wobei die ersten mechanischen Verbindungsmittel (202, 203) die lösbare Verbindung des Positionierungs- und Führungsteils (2A) in Bezug auf ein Antriebssystem (4), das mindestens ein Paar von Antriebsrollen (40, 41) umfasst, erlauben, derart, dass der erste Abschnitt (110a) des Leitungselements (1) zur Abgabe von Behandlungsdosen mittels des Positionierungs- und Führungsteils (2A) zwischen die/den Antriebsrollen (40, 41) positioniert und geführt werden kann, um das Leitungselement (1) zur Abgabe von Behandlungsdosen in dessen Längsrichtung zumindest in einer ersten Richtung (R) anzutreiben.

11. Anordnung nach einem der vorhergehenden Ansprüche, wobei das Positionierungs- und Führungsteil (2A), das Leitungselement (1) zur Abgabe von Behandlungsdosen, gegebenenfalls die flexible Führung (3) und gegebenenfalls der Speicherträger (2B) steril sind und in einer luftdichten Verpackung angeordnet sind, und/oder wobei die ersten mechanischen Verbindungsmittel (202, 203) eine schnelle und werkzeuglose Verbindung des Positionierungs- und Führungsteils (2A) erlauben.

12. Vorrichtung zur endovenösen Behandlung, mit:
einer Anordnung nach einem der Ansprüche 1 bis 9 und
einem Antriebssystem (4), das bevorzugt motorisiert ist, welches Antriebsmittel (40, 41) umfasst,
wobei die Vorrichtung weiterhin zweite mechanische Verbindungsmittel (42, 43) umfasst, die so ausgelegt sind, dass sie mit den ersten mechanischen Verbindungsmitteln (202, 203) des Positionierungs- und Führungsteils (2A) zusammenarbeiten, um die lösbare Verbindung des Positionierungs- und Führungsteils (2A) in Bezug auf das Antriebssystem (4) zu ermöglichen, derart, dass der erste Abschnitt (110a) des Leitungselements (1) zur Abgabe von Behandlungsdosen so positioniert ist, dass er mit den Antriebsmitteln des Antriebssystems (4) in Verbindung sein kann, und dass die Antriebsmittel des Antriebssystems (4) den Antrieb des Leitungselements (1) zur Abgabe von Behandlungsdosen in dessen Längsrichtung zumindest in einer ersten Richtung (R) erlauben, bevorzugt mit einer Bewegung des Leitungselements (1) zur Abgabe von Behandlungsdosen in dessen Längsrichtung relativ zu den Antriebsmitteln.

13. Vorrichtung zur endovenösen Behandlung nach Anspruch 12, wobei die Antriebsmittel des Antriebssystems (4) mindestens ein Paar Antriebsrollen (40, 41) umfassen und die zweiten mechanischen Verbindungsmittel (42, 43) dazu ausgelegt sind, mit den ersten mechanischen Verbindungsmitteln (202, 203) des Positionierungs- und Führungsteils (2A) zusammenzuwirken, um die Positionierung des ersten Abschnitts (110a) des Leitungselements (1) zur Abgabe von Behandlungsdosen zwischen den Antriebsrollen (40, 41) zu ermöglichen.

14. Vorrichtung zur endovenösen Behandlung nach einem der Ansprüche 12 bis 13, wobei die Antriebsmittel des Antriebssystems (4) einen Antrieb des Leitungselements (1) zur Abgabe von Behandlungsdosen mittels Reibung erlauben.

15. Vorrichtung zur endovenösen Behandlung nach einem der Ansprüche 12 bis 14, weiterhin umfassend eine weitere Quelle für Behandlungsdosen (L), die mit dem hinteren Ende des Leitungselements (1) zur Abgabe von Behandlungsdosen verbunden werden kann, und wobei bevorzugt das Antriebssystem (4) und die Quelle für Behandlungsdosen (L) einen integralen Bestandteil einer gleichen monolithischen Einheit (E) bilden.

16. Vorrichtung zur endovenösen Behandlung nach Anspruch 15, wobei das Leitungselement (1) zur Abgabe von Behandlungsdosen eine optische Faser ist und die Quelle für Behandlungsdosen (L) eine elektromagnetische Strahlungsquelle ist.

17. Verfahren zur Herstellung einer Vorrichtung zur endovenösen Behandlung nach einem der Ansprüche 12 bis 16, wobei das Positionierungs- und Führungsteil (2A) ablösbar in Bezug auf das Antriebsmittel (4) derart verbunden wird, dass ein erster Abschnitt (11a) des Leitungselements zur Abgabe von Behandlungsdosen so positioniert wird, dass dieser mit den Antriebsmitteln des Antriebssystems in Verbindung sein kann und zumindest in einer ersten Antriebsrichtung angetrieben werden kann.

## Claims

1. Endovenous treatment assembly comprising a wire element (1) for delivering treatment doses, which is flexible and capable of being inserted, over a part of its length, longitudinally into a vein (V), and a positioning and guiding part (2A), which is integral with the wire element (1) for delivering treatment doses so as to allow the manipulation of the wire element (1) for delivering treatment doses by means of this positioning and guiding part (2A), said positioning and guiding part (2A) comprising at least a first guiding mean (200a, 200b, 200c) for guiding in translation a first portion (110a) of the wire element (1) for delivering treatment doses in the direction of its length, the wire element (1) for delivering treatment doses being able to slide in the direction of its length relative to said first guiding mean (200a, 200b, 200c) of said positioning and guiding part (2A), being guided in translation by said first guiding mean (200a, 200b, 200c), and said positioning and guiding part (2A) comprising first mechanical assembly means (202, 203) allowing its removable mounting relative to drive means of a drive system (4), so that said first portion (110a) of the wire element (1) for delivering treatment doses can be positioned and guided by means of this positioning and guiding part (2A) so as to be able to be engaged with drive means of the system drive (4) for driving the wire element (1) for delivering treatment doses in the direction of its length at least in a first direction (R).

2. Assembly according to claim 1, wherein said first portion (110a) of the wire element (1) for delivering treatment doses is accessible or can be made accessible.

3. Assembly according to any one of claims 1 or 2, in which said first portion (110a) of the wire element (1) for delivering treatment doses can be positioned and guided by means of the positioning and guiding part (2A) so as to be able to be engaged with drive means of the drive system (4) for driving the wire element (1) for delivering treatment doses in the direction of its length at least in one first direction (R), with a displacement of the wire element (1) for delivering treatment doses in the direction of its length relative to said drive means.

4. Assembly according to any one of the preceding claims comprising second guide means (201) of a second portion of the wire element (1) for delivering treatment doses, and in which the second guide means (201) guide in translation this second portion of the wire element (1) for delivering treatment doses by making it perform at least a quarter turn and preferably at least half a turn and/or form an integral part of the positioning and guiding part (2A).

5. Assembly according to claim 4, in which the wire element (1) for delivering treatment doses comprises a third portion (110c), which extends to the rear end of the wire element (1) for delivering treatment doses, and which is not integral with the positioning and guiding part (2A) or which is integral with the positioning and guiding part (2A) but can be separated from this positioning and guiding part (2A).

6. Assembly according to any one of the preceding claims, in which the positioning and guiding part (2A) is a single part, and more particularly a molded part or in which the positioning and guiding part (2A) is constituted by a monolithic assembly.

7. Assembly according to any one of the preceding claims, comprising a flexible guide (3) threaded on a front portion of the wire element (1) for delivering treatment doses, said wire element (1) for delivering treatment doses being able to slide longitudinally with respect to the flexible guide (3), and preferably wherein the rear end (31) of the flexible guide (3) is integral with the positioning and guiding part (2A), so as to axially block the rear part of the flexible guide (3) at least in the first drive direction (R) of the wire element (1) for delivering treatment doses, and preferably also in the direction (F) opposite to the drive direction (R) of the wire element (1) for delivering treatment doses.

8. Assembly according to any one of the preceding claims, comprising a storage support (2B), on which is wound all or part of the front portion of the wire element (1) for delivering treatment doses which extends from the front end of the wire element (1) for delivering doses to said first portion (110a) of the wire element (1) for delivering treatment doses, and preferably in which a flexible guide (3) threaded on a said front portion of the wire element (1) for delivering treatment doses, said wire element (1) for delivering treatment doses being able to slide longitudinally with respect to the flexible guide (3) and the flexible guide (3) and the part of the wire element (1) for delivering treatment doses threaded into the flexible guide (3) being wound on the storage support (2B).

9. Assembly according to any one of the preceding claims, in which the wire treatment dose delivery element (1) is an optical fiber.

10. Assembly according to any one of the preceding claims, in which the first mechanical assembly means (202, 203) allow the removable mounting of the positioning and guiding part (2A) relative to a drive system (4) which comprises at least one pair of drive rollers (40, 41), so that said first portion (110a) of the wire element (1) for delivering treatment doses can be positioned and guided by means of this positioning and guiding part (2A) between the drive rollers (40, 41), for driving the wire element (1) for delivery of treatment doses in the direction of its length at least in a first direction (R)

11. Assembly according to any one of the preceding claims, in which the positioning and guiding part (2A), the wire element for delivering treatment doses (1), if necessary the flexible guide (3), and if necessary the storage support (2B), are sterile and placed in an airtight package and/or in which the first mechanical assembly means (202, 203) allow rapid and tool-free assembly of the positioning and guiding part (2A).

12. Endovenous treatment device comprising an assembly as claimed in any one of claims 1 to 9 and a drive system (4), preferably motorized, which comprises drive means (40, 41), the device further comprising second mechanical assembly means (42, 43) adapted to cooperate with the first mechanical assembly means (202, 203) of the positioning and guiding part (2A), so as to allow said removable mounting of the positioning and guiding part (2A) relative to the drive system (4), so that said first portion (110a) of the wire element (1) for delivering treatment doses is positioned so as to be able to be engaged with drive means of the drive system (4) and the drive means of the drive system (4) allow the drive of the wire element (1) for delivering treatment doses in the direction of its length at least in a first direction (R), with preferably a displacement of the wire element (1) for delivering treatment doses in the direction of its length relative to said drive means.

13. Endovenous treatment device according to claim 12, in which the drive means of the drive system (4) comprise at least one pair of drive rollers (40, 41) and the second mechanical assembly means (42, 43) are adapted to cooperate with the first mechanical assembly means (202, 203) of the positioning and guiding part (2A), so as to allow the positioning of the first portion (110a) of the wire element (1) for delivering treatment doses between the drive rollers (40, 41).

14. Endovenous treatment device according to any one of claims 12 to 13, in which the drive means of the drive system (4) allow friction drive of the wire element (1) for delivering treatment doses.

15. Endovenous treatment device according to any one of claims 12 to 14, further comprising a source of treatment doses (L) able to be connected to the rear end of the wire element (1) for delivering treatment doses, and preferably in which the drive system (4) and the treatment dose source (L) are an integral part of the same monolithic assembly (E).

16. Endovenous treatment device according to claim 15, in which the wire element (1) for delivering treatment doses is an optical fiber and the treatment dose source (L) is a source of electromagnetic radiation.

17. Method for preparing an endovenous treatment device according to any one of claims 12 to 16 during which the positioning and guiding part (2A) is removably mounted relative to the drive system (4) so that a first portion (11a) of the wire element for delivering treatment doses is positioned so as to be able to be engaged with the drive means of the system of drive and can be driven at least in a first drive direction.
